# EUROPEAN PATENT APPLICATION

(11) **EP 4 566 615 A1**
(43) Date of publication of application: **11.06.2025**
(21) Application number: 24216999.3
(22) Date of filing: 03.12.2024
(51) Int. Cl.: A61K 35/12, A61K 38/18, A61L 27/54, A61P 19/08

(54) **JOINT STRUCTURE RECONSTRUCTION INDUCER AND THE METHOD OF USING THE SAME AND THE USE THEREOF**

(30) Priority: 04.12.2023 CN 202311642249
(71) Applicant: Guilin Medical University, Guilin, Guangxi (CN)
(72) Inventor: LIU, Yingqin, Guilin (CN); LI, Boyu, Guilin (CN); WANG, Zijian, Guilin (CN); SUN, Xin, Guilin (CN)
(74) Representative: Latscha Schöllhorn Partner AG

(57) **Abstract**

The present invention belongs to the in-situ regeneration induction in the field of regenerative medicine, especially the reconstruction of joint-like structures in mammals.

## Description

### TECHNICAL FIELD

The present invention belongs to the in-situ regeneration induction in the field of regenerative medicine, especially the reconstruction of joint-like structures in mammals. The present invention includes joint-like structures that regenerate in adult murine digits, which are regenerations in mammals.

### BACKGROUND

Lower organisms have strong regenerative capacity. For example, amphibians such as lizards, salamanders and frogs can fully regenerate their front and back limbs after amputation. However, mammals have poor regenerative capacity. For example, the distal phalanges (Phalange 3, P3) of human and murine have only incomplete regenerative capacity (*i.e.,* it can regenerate after distal amputation but not upon proximal amputation), and the other phalanges such as middle phalanges (Phalange 2, P2) as well as the upper and lower limbs have no regenerative capacity.

Joint amputation is an injury to the joint structure, and is an abnormality of joint wherein the joint element(s) is(are) defective. Joint amputation is different from common joint injuries. Common joint injuries, such as osteoarthritis, micro-drilling on the joint, etc., have intact joint structures with no missing of joint elements. The repair of joint structure injuries is a difficult problem that has not yet been solved. Mammalian joints cannot regenerate after amputation, and research on regeneration after joint amputation is generally conducted in amphibians which have regenerative capacity [Tsutsumi, Rio et al., "Functional joint regeneration is achieved using reintegration mechanism in Xenopus laevis." Regeneration (Oxford, England) vol. 3,1 26-38. 6 Jan. (2016), doi:10.1002/reg2.49; Tsutsumi, Rio et al., "Reintegration of the regenerated and the remaining tissues during joint regeneration in the newt Cynops pyrrhogaster." Regeneration (Oxford, England) vol. 2,1 26-36. 8 Apr. 2015, doi:10.1002/reg2.28]. So far, there is only one report on regeneration study of complete missing of j oint elements in mammals, which is murine digital joints regeneration induced by BMP9: amputation of the murine digit at P2, with complete missing of P3 and the sesamoid bones in the terminal joint of the murine digits and partial missing of the P2 element. BMP9 can induce a joint-like structure at the P2 stump [Yu, Ling et al. "BMP9 stimulates joint regeneration at digit amputation wounds in mice." Nature communications vol. 10,1 424. 5 Feb. (2019), doi:10.1038/s41467-018-08278- 412].

There is therefore an unmet need in the art for the inducers for joint structure reconstruction, particularly where joint element(s) is(are) defective. The present invention aims to solve the technical problem.

### DISCLOSURE OF INVENTION

The cartilages of the murine digital joints are hyaline cartilages, which are avascular connective tissue, and the main component thereof is cartilage matrix. The cartilage matrix has a dense texture and can bear pressure; chondrocytes are present in the cartilage matrix. The chondrocytes of the hyaline cartilage are generally in a circular or biconvex lens shape, with a distinct contrast between the dark nucleus and the white outside the nucleus, which looks like an eyeball and is easy to be identified. Especially, the enlarged hypertrophic chondrocytes are easier to identify. Cartilage can differentiate into bone through endochondral ossification.

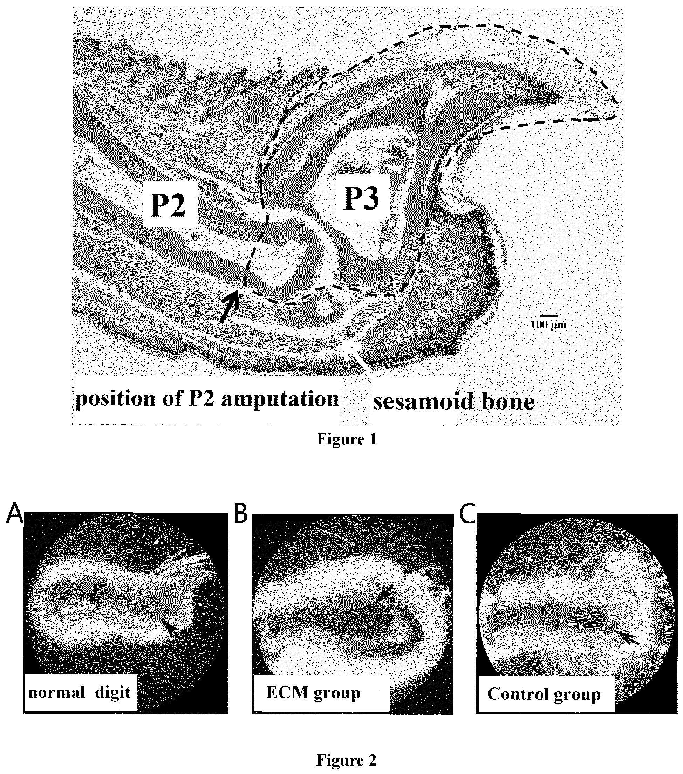

In the P2 amputation regeneration study, we used for the first time the P2 amputation model with the retained soft tissue (Figure 1. normal murine digit): the murine digit was cut at P2, and the P3 and the distal end of P2 were removed, with retaining the soft tissue as much as possible. We have found in this model that extracellular matrix (ECM) promotes bone regeneration of P2 (patent application PCT/CN2017/102270, *Inducer for Synchronous Regeneration of Bone and Soft Tissue, Preparation Method Therefor and Use Thereof*). The P2 amputation model was originally used as a bone regeneration model, but from the perspective of joint regeneration, this model is also a murine digit terminal joint amputation model: the murine digit terminal joint consists of three joint elements, *i.e.,* P2, sesamoid bone, and P3; in the P2 amputation model with retaining soft tissue, the P3 element in the joint is completely missing, the P2 element is partially missing/partially retained, and the sesamoid bone is retained.

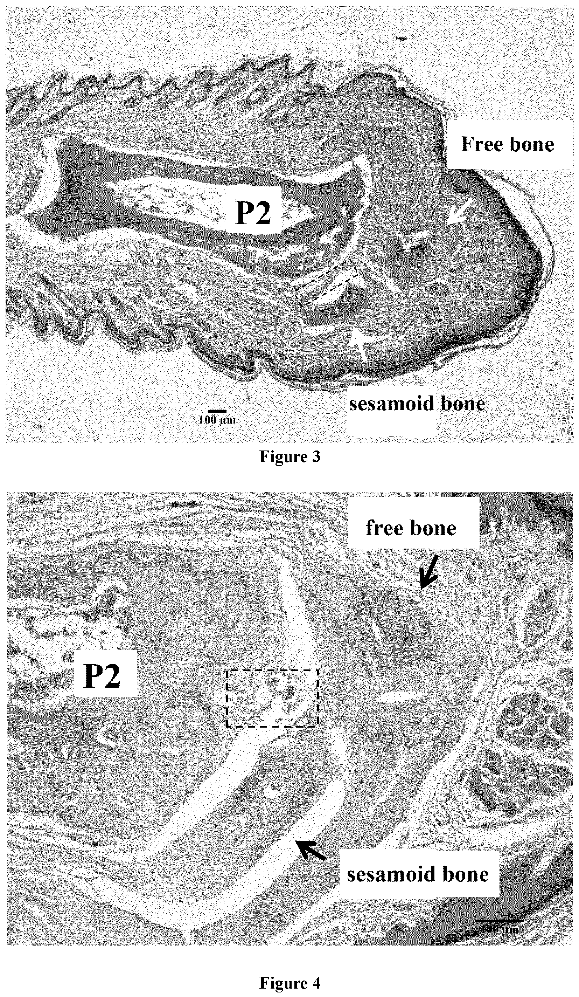

In the ECM-induced regeneration of P2 amputation, it was surprisingly found that free bone formed in the ECM group, whereas no free bone appeared in the control group (Figure 2). Moreover, it was found that the retained sesamoid bone could form joint-like structures with P2 in both the control group and the ECM group, and the free bone in the ECM group can also form joint-like structures with P2. Thus, the present inventors have surprisingly found that: (1) the ECM can induce the formation of free bone; (2) the sesamoid bone can induce a joint-like structure at the stump of P2; (3) free bone can merge with sesamoid bone, and the merged bone does not affect the formation and maintenance of joint-like structures. In the above studies, the ECM was implanted into the murine digit immediately after the amputation. Further studies show that the chance of formation of free bone may increase when the ECM implantation is performed after the healing of the amputation wound (Figure 3-7).

The inventors have also found that: where the joint element(s) is(are) defective, the retained joint element(s) may reform a joint-like structure with the stump of the partially missing joint element(s). Namely, in the P2 amputation model, the sesamoid bone can reconstruct a joint-like structure with P2 (among the three elements of the murine digit terminal joint, the P3 element is completely missing, the sesamoid bone element is retained, and the P2 element is partially missing). The inventors have also found that: where the joint element(s) is(are) defective, ECM granules can form free bone at the site of defect, which can form joint-like structures with the retained joint element (sesamoid bone) and also with the stump of the joint element (P2). The free bone may merge with the retained joint element (sesamoid bone), and the merged bone dose not merge with the partially missing joint element (P2), so that the joint-like structure is maintained and is stable for a long period of time. In the P3 removal model, ECM granules can regenerate P3 bone, which forms joint structures with sesamoid bone and P2, respectively.

In one aspect, the present invention relates to the following embodiments:
Embodiment 1. the main ingredient of the inducer is ECM, characterized in that the ECM is a product derived from animal tissues and organs as raw material, and the ECM includes but is not limited to: extracellular matrix from small intestine, trachea, bladder, or bone, or extracellular matrix derived from non-animal tissue or organs.
Embodiment 2. the main ingredient of the inducer is ECM, characterized in that the ECM is the product in Embodiment 1 which is treated by physical or chemical methods, including but not limited to: lyophilizing, pulverizing, degradation, cross-linking, gelation, 3D printing and the like.
Embodiment 3. the inducer further comprises adjuvants including, but not limited to: pure water or neutral buffer; inorganic salts or inorganic salt solutions containing calcium, phosphorus, magnesium, and the like.
Embodiment 4. the inducer is a drug for external use, which works by implantation into the lesion site of a tissue and is completely absorbed during the regeneration.
Embodiment 5. the main ingredient of the inducer is ECM, characterized in that the ECM refers to any of Embodiments 1 or 2.

In another aspect, the present invention relates to an inducer comprising an extracellular matrix (ECM). In some embodiments, the inducer of the invention comprises extracellular matrix as main ingredient. In some embodiments, the inducer of the invention comprises extracellular matrix as main active ingredient. In some embodiments, the inducer of the invention comprises extracellular matrix as the sole active ingredient.

In some embodiments, the inducer of the invention is useful for inducing joint reconstruction in a subject. In some embodiments, the inducer of the invention is useful for inducing joint reconstruction in a subject where joint element(s) is(are) defective. In some embodiments, the inducer of the invention is useful for inducing joint reconstruction in a subject where at least one joint element is completely missing.

In another aspect, provided is the use of the inducer of the invention for use in induing joint reconstruction in a subject.

In another aspect, provided is the use of the inducer of the invention in the manufacture of a medicament for inducing j oint reconstruction in a subject.

In another aspect, provided is a method of achieving joint reconstruction in a subject, comprising contacting the inducer of the invention with the lesion site of the joint in the subject. In some embodiments, the present invention relates to a method of achieving joint reconstruction in a subject, comprising contacting the inducer of the invention with the lesion site of the joint in the subject, ECM shaping, and delayed implantation.

In some embodiments, the joint reconstruction is a joint reconstruction in the case of joint element defect, e.g., in the case of partial or complete missing of joint element(s). Preferably, the joint reconstruction is a joint reconstruction in the case of complete missing of at least one joint element (e.g., P3). In some embodiments, the joint element may be any joint element, such as a P2 element, a P3 element, a sesamoid bone or the like.

In some embodiments, the joint element defect is a freshly generated joint element defect, e.g., immediately or shortly after a joint amputation (e.g., within 1, 2, 3, 4, 5, 6 days or within 1 week, etc.). In some embodiments, the joint element defect is an old joint element defect. In some embodiments, the joint element defect is a joint element defect that is freshly generated, e.g., by joint amputation, at the j oint site of an old j oint element defect.

In some embodiments, the extracellular matrix is obtained from an animal tissue and organ, including but not limited to extracellular matrices obtained from small intestine, trachea, bladder, bone. In some embodiments, the extracellular matrix is of non-animal tissue or organ origin.

In some embodiments, the extracellular matrix is obtained by physical or chemical treatment, including but not limited to: lyophilizing, extruding, pulverizing, degradation, cross-linking, gelation, 3D printing and other shaping treatment.

In some embodiments, the inducer of the invention further comprises adjuvants including, but not limited to, inorganic salts such as phosphate, acetate, trehalose, hydroxyapatite micronised, glucose, sorbitol, purified water, ethanol, inorganic salt solutions such as phosphate buffer, acetate buffer, citrate buffer. In some embodiments, the adjuvant is hydroxyapatite micronised and/or an inorganic salt or an inorganic salt solution may be optionally added.

In some embodiments, the inducer of the invention further comprises hydroxyapatite micronised as an adjuvant. In some embodiments, the dry weight ratio of hydroxyapatite micronised to ECM is 100:1∼1: 10000, e.g., 10:1∼1:200, 5:1∼1:100, 2:1∼1:50, 1:10∼1:50.

In some embodiments, the adjuvant is a liquid diluent. The liquid diluent may optionally be supplemented with inorganic salts or other osteogenesis-promoting components including, but not limited to: inorganic salts containing calcium, phosphorus, magnesium, and the like. The liquid diluent includes, but is not limited to, pure water, phosphate buffer, acetate buffer, citrate buffer, etc., preferably pure water or neutral buffer, more preferably phosphate buffer containing 5% trehalose (preferably pH = 7.2).

In some embodiments, the inducer of the invention further comprises additional components, such as one or more selected from the group consisting of inorganic salts (e.g., inorganic salts containing calcium, phosphorus, magnesium, etc.), acetate, citrate, trehalose, hydroxyapatite micronised, glucose, sorbitol, or other osteogenesis-promoting elements.

In some embodiments, the inducer of the invention consists of extracellular matrix and adjuvant(s).

In some embodiments, the regeneration-inducer of the invention consists of extracellular matrix and a liquid diluent.

In some embodiments, the inducer of the invention consists of extracellular matrix.

In some embodiments, the inducer of the invention is in the form of granules (e.g., microparticles), e.g., formed by extruding the powder or the powder mixture of granule components into granules. The particle size of the granules is not particularly limited, as long as it can be suitably implanted into a joint lesion site of a subject. For example, the granules may have a particle size distribution in the range of 1µm-10mm, such as 10µm, 50µm, 100µm, 200µm, 300µm, 400µm, 500µm, 600µm, 700µm, 800µm, 900µm, 1mm, 2mm, 3mm, 4mm, 5mm, 6mm, 7mm, 8mm, 9mm, 10mm or a range formed by any two therefrom.

The method for preparing the granules is not particularly limited. In some embodiments, the inducer granules are prepared by dry granulation or wet granulation. In dry granulation, the powders of all the granule components may be uniformly mixed and then directly extruded into granules. In wet granulation, the powder or powder mixture of the granule components (e.g., ECM and/or one or more of the additional components) may be wetted with a liquid (e.g., water, ethanol, solution or suspension), extruded into granules, and then dried. For wet granulation, the additional components may be added in the powder mixture to be wetted, or may be added (e.g. dissolved or suspended) in the liquid for wetting; or a part of the additional components are added in the powder mixture to be wetted and the rest is added (e.g. dissolved or suspended) in the liquid for wetting. In some embodiments, the liquid for wetting is a buffer optionally comprising nutrients or other osteogenesis-promoting elements, such as a buffer at pH6-8, including but not limited to a phosphate buffer, an acetate buffer, or a citrate buffer. In some embodiments, the buffer comprises 5% trehalose.

In some embodiments, the inducer of the invention is shaped into the shape of a bone to be regenerated, such as the shape of a P3 element or the shape of a missing portion of a P2 element.

In some embodiments, the regeneration-inducer of the invention is for external use, which works by implantation into the lesion site of a tissue and is partially or completely absorbed during the regeneration process.

In some embodiments, the inducer of the invention is implanted into a joint lesion site of a subject immediately or soon after the joint element defect in the subject (i.e., normal implantation). In some embodiments, the normal implantation of the invention is performed within 1 week, e.g., within 1, 2, 3, 4, 5, 6 or 7 days, from the joint element defect in the subject.

In some embodiments, the inducer of the invention is implanted into a joint lesion site of a subject after a period of time (e.g., a period of 1 week or more) from the joint element defect in the subject (i.e., delayed implantation). In some embodiments, the delayed implantation of the invention is performed at any time point during the period of Week 2-12, or Week 2-8, or Week 2-4 from joint element defect in the subject. In some embodiments, the delayed implantation of the invention is performed at any time point during Week 2, Week 3, or Week 4 from the joint element defect in the subject. In some embodiments, the delayed implantation of the invention is performed after the healing of wound at the j oint defect site.

In some embodiments, the inducer of the invention may be implanted by both normal implantation and delayed implantation. That is, the inducer of the invention is implanted into the joint defect site of the subject immediately or soon after the joint element defect occurs in a subject (e.g., within 1 week, e.g., within 1, 2, 3, 4, 5, 6 or 7 days) (normal implantation), and is implanted again after a period of time (e.g., a period of 1 week or more, preferably at any time point during the period of Week 2-12, Week 2-8, or Week 2-4 from the joint element defect in the subject) (delayed implantation).

In another aspect, provided is a kit of parts, including two containers, wherein the first container comprises the inducer of the invention for implantation into a joint defect site of a subject immediately or soon after joint element defect occurs in the subject (normal implantation), and the second container comprises the inducer of the invention for implantation into a joint defect site of a subject after a period of time (e.g., a period of 1 week or more, preferably at any time point during the period of Week 2-12, Week 2-8, or Week 2-4) from the joint element defect in the subject, e.g., after healing of wound at the joint defect site (delayed implantation).

In some embodiments, the subject is a mammal, bird, fish, or reptile. In some embodiments, the subject is a mammal. In some embodiments, the subject is selected from the group consisting of cats, dogs, sheep, goats, cattle, horses, pigs, mice, rats and guinea pigs. In some embodiments, the subject is a human.

The effect of the inducer of the invention is as follows: in the mouse P2 amputation model with the retained soft tissue, the inducer can induce the formation of free bone at the digit stump. Free bone and P2 can form joint-like structures that are stably maintained and exists for a long time. The free bone and the sesamoid bone can merge into one bone, and the joint-like structure formed by the merged bone and the P2 is stably maintained and exists for a long time.

### Definition

It shall be understood that the term used herein is for the purpose of illustrating the particular embodiments of the invention only and is not intended to be limiting. Unless defined otherwise, all the technical and scientific terms used herein have the same meaning as commonly understood by one of ordinary skill in the art to which this present invention belongs.

The term "in-situ regeneration" refers to the process in which the entire organism, tissue, or organ is partially lost or damaged due to trauma, and the structures that are identical or similar in morphology and function to the missing or damaged part are grown on the basis of the remaining part. This repair process is called in-situ regeneration.

The term "joint element" refers to the individual bones in the joint, which is one joint element, including the articular cartilage on the surface of the bones. For example, the phalangeal terminal joint of mouse consists of three joint elements, namely P2, P3, and the sesamoid bone.

The term "joint element defect" or "joint lesion" or "joint damage" includes one or both of "partial missing of joint element(s)" and "complete missing of joint element(s)", which both refer to the structural defect of a joint. It is different from the injuries with intact structure, e.g., by micro-drilling. As in the P2 amputation wound model, P3 was totally removed, so that P3 is a complete missing of joint element; where the distal portion of P2 is removed but the proximal portion is remained, P2 is a partial missing of joint element.

The term "old joint element defect" means that the joint element defect occurs for more than 4 weeks, e.g., more than 1 month, 4 months, 8 months, 1 year, 2 years, 3 years, 4 years, 5 years, 10 years, 20 years, 30 years, 40 years or more.

The term "freshly generated joint element defect" means that the joint element defect occurs within 4 weeks, e.g., within 1, 2, 3, 4, 5, 6 days or within 1, 2, 3 or 4 weeks. In this context, "freshly generated joint element defect" also includes a joint element defect that is freshly generated at the joint site of an old joint element defect, such as by a joint amputation.

The term "joint structure" and "joint-like structure" are used interchangeably and refer to a structure consisting of the terminals of two adjacent bone and the space therebetween, both bone terminals being covered by a layer of cartilage.

The term "free bone" refers to regenerated bone that is not connected to the original bone and is encapsulated by connective tissue.

The term "regenerated P3 bone" refers to free bone induced by ECM in the P3 removal model. In the P3 removal model, the regenerated free bone is usually only one piece, and the characteristics such as position and shape of the free bone are close to those of the P3 bone when the free bone dose not merge with the sesamoid bone, so that the free bone is called the regenerated P3 bone.

The terms "joint reconstruction", "joint structure reconstruction" and "joint-like structure reconstruction" are used interchangeably and refer to the reconstruction of a joint-like structure at the site of a joint defect in a subject having a complete or partial missing of joint element(s).

The terms "extracellular matrix" and "ECM" and the like are used interchangeably herein and mean, in a narrow sense, collagen-rich material found between the cells of animal tissue and used as a structural element in the tissue. It typically comprises a complex mixture of polysaccharides and proteins secreted by the cells. The extracellular matrix can be isolated and treated in various ways. The extracellular matrix (ECM) can be obtained by isolating from a tissue and organ of an animal, including mammals, e.g., domestic or farm animals, such as cats, dogs, sheep, goats, cattle, horses and pigs, and primates; birds; laboratory animals, such as mice, rats and guinea pigs; the animal tissue and organs include but are not limited to small intestine, trachea, bladder, bone, small intestine submucosa, stomach submucosa, bladder submucosa, tissue mucosa, dura mater, liver basement membrane, pericardium, or other tissue. After isolation and treatment, it is commonly referred to as the extracellular matrix or ECM. In a broad sense, the extracellular matrix also includes extracellular matrices derived from non-animal tissue or organs, such as artificial extracellular matrices, extracellular matrices derived from cells cultured *in vitro,* and the like. Preferably, the extracellular matrix may be prepared according to the method described in Agrawal V, Johnson SA, Reing J. Epimorphic regeneration approach to tissue replacement in adult mammals. [J]. Proc Natl Acad Sci, 2010, USA 107:3351-3355.

The terms "inducer" and "regeneration inducer" are used interchangeably and refer to a composition that induces tissue and/or organ regeneration in a subject, in particular, a composition that induces joint reconstruction.

The term "adjuvant" refers to the substance other than an active ingredient (e.g., ECM), which is/are added to a pharmaceutical formulation during the preparation or formulation. The adjuvant may be a liquid, solid or gas, and may be a single substance or a mixture of multiple substances. The adjuvants include filler, stabilizer, antibiotic, antioxidant, antiseptic, diluent (including liquid diluent), etc. The adjuvants of the invention are known to those skilled in the art and include, but are not limited to, hydroxyapatite micronised, phosphate, acetate, trehalose, glucose, sorbitol, purified water, ethanol, phosphate buffer, acetate buffer, citrate buffer, and the like. For information on more excipients, see the references in the art, for example, Mingsheng Luo, Tianhui Gao, pharmaceutical excipients, 2nd Edition, Sichuan scientific and technical Press.

The term "liquid diluent" refers to a liquid used to dilute or wet the ECM, for example an organic or inorganic solvent such as water, ethanol or mixtures thereof. The liquid diluent may optionally be supplemented with inorganic salts or other osteogenesis-promoting elements including, but not limited to: inorganic salts containing calcium, phosphorus, magnesium, and the like. The liquid diluent of the invention includes, but is not limited to, pure water, phosphate buffer, acetate buffer, citrate buffer, etc., preferably pure water or neutral buffer, such as phosphate buffer containing 5% trehalose (pH = 7.2).

The terms "subject" and "patient" are used interchangeably and include mammals, e.g., domestic or farm animals, such as cats, dogs, sheep, goats, cattle, horses and pigs, humans and primates; birds; laboratory animals such as mice, rats and guinea pigs; fish; reptile, preferably a human.

### Administration

The inducers of the invention may be formulated in any suitable dosage form and may be administered by any suitable route. The inducer of the invention may be formulated into a solution, suspension, emulsion, lyophilized preparation and the like for injection, into granules, gels, ointments, creams, suppositories, patches and the like for topical administration, and into aerosols, sprays, powders and the like for topical administration. Generally, the preferred mode of administration is direct topical administration at the tissue defect. More preferably, the inducer of the invention works by implantation into the lesion site of a tissue and is completely absorbed during the regeneration process.

### BRIEF DESCRIPTION OF THE DRAWINGS

Figure 1 shows normal murine digit, wherein black arrows indicate the position of P2 amputation, white arrows indicate the sesamoid bones, and within the dashed box is the parts that will be removed by digit amputation.
Figure 2 shows normal murine digit (A) and a comparison between ECM normal implantation (B) on Day 20 and the control group (C) in the P2 amputation model (Example 1). Here, the normal implantation of ECM granules is conducted immediately after the digit amputation. The arrows in A and C indicate the sesamoid bone, and the arrow in B indicates the free bone.
Figure 3 shows photographs of ECM delayed implantation on Day 21 in the P2 amputation model (Example 2). Delayed implantation of ECM granules is conducted at week 2 after amputation. White arrows point to the sesamoid and free bones, respectively, and within the dashed box is soft tissue (connective tissue) in the bone space between P2 and the sesamoid bone.
Figure 4 shows photographs of ECM delayed implantation on Day 33 in the P2 amputation model (Example 2). Black arrows point to the sesamoid and free bones, respectively, and within the dashed box is the soft tissue (connective tissue) in the joint space.

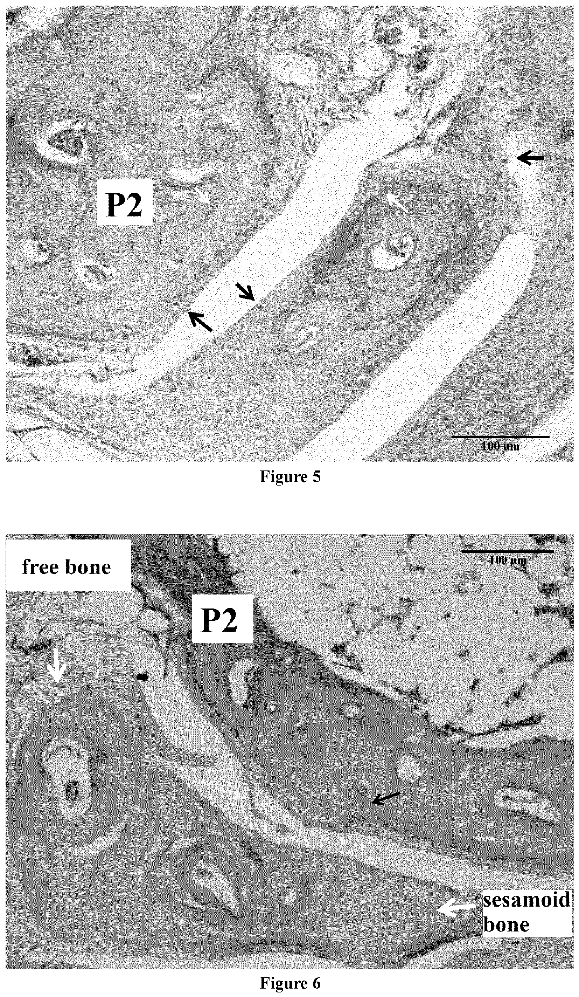

Figure 5 is a photograph of ECM delayed implantation on Day 33 in the P2 amputation model (enlargement of Figure 4). The black arrows indicate chondrocytes. Chondrocytes are distributed primarily in cartilage, and also in the soft tissues adjacent to cartilage. The white arrows indicate the edges of the cartilage.
Figure 6 is a photograph of ECM delayed implantation on Day 65 in the P2 amputation model (Example 2). White arrows point to the sesamoid bone and the free bone, respectively, and black arrows point to the edge of the regenerated cartilage. The edge line of the regenerated cartilage is an irregular curve due to uneven thickness of the regenerated cartilage layer.

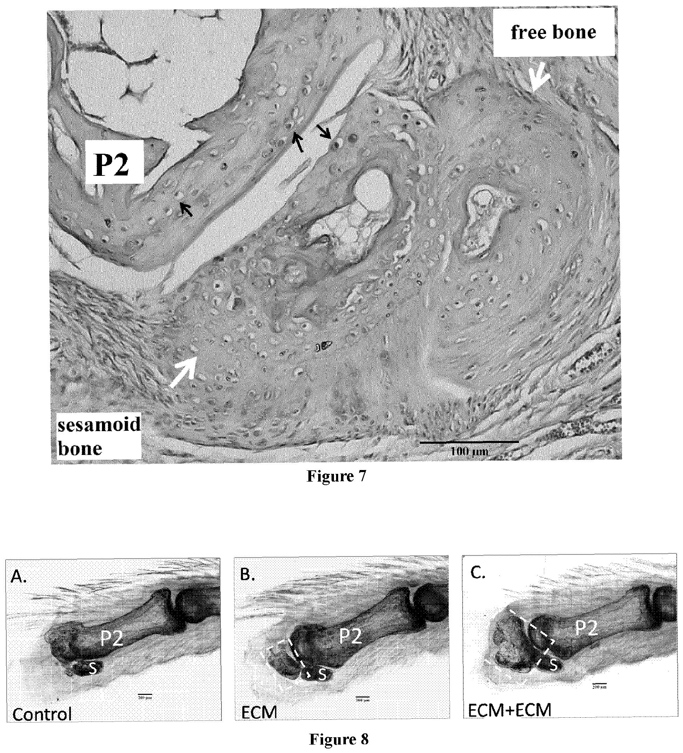

Figure 7 is a photograph of ECM delayed implantation on Day 98 in the P2 amputation model (Example 2). White arrows point to the sesamoid bone and free bone, respectively, and black arrows point to chondrocytes.
Figure 8 shows photographs of the control group (A) and the ECM normal implantation group (B) and the ECM normal implantation + delayed implantation group (C) (Examples 3 and 4) in the P3 ablation model, wherein "s" is sesamoid bone and regenerated P3 bone is within the white dashed box. Scale =200 microns.

### EXAMPLES

The following examples are intended to illustrate the present invention in more details, but should not be construed as limiting the scope of the invention as defined by the claims.

### Example 1: ECM normal implantation in P2 amputation model

Preparation of ECM. Fresh pig bladder was taken and ECM lyophilized powder was prepared according to literature methods [Agrawal V, Johnson SA, Reing J. Epimorphic regeneration approach to tissue replacement in adult mammals. [J]. Proc Natl Acad Sci, 2010, USA 107:3351-3355]. The ECM granules were prepared by directly extruding the ECM lyophilized powder into granules, or by wetting the ECM lyophilized powder with purified water, extruding into granules and drying in air (about 0.125 µl/granule purified water). The ECM granules had a dry weight of about 0.25 mg/granule and a particle size of about 0.5 mm.

P2 amputation model and use of inducer: adult mice were anesthetized and P2 amputation was performed on the second digit/fourth digit of the hind foot with remaining the soft tissue: that is, the murine digit was amputated at the P2 phalange, and the amputated bone tissue was removed (*i.e.,* P3 and the distal end of P2 were removed), leaving as much soft tissue as possible. ECM granules were implanted (1 granule/digit) immediately after P2 amputation in ECM group, and no implantation is conducted after P2 amputation in control group, and no treatment thereafter.

On Day 20 after implantation, free bone formation was observed in the ECM group, whereas the control group had no free bone formation throughout (Figure 2).

### Example 2: ECM delayed implantation in P2 amputation model

Adult mice were subjected to P2 amputation (see Example 1) and were not treated thereafter. At Week 2 after P2 amputation, the mice were again anesthetized, and the skin at the end of the digit was incised without damaging the bone tissue. ECM granules were implanted in through the skin incision in the ECM group, no implantation is performed in the control group after the skin incision. ECM granules were prepared by directly extruding the ECM lyophilized powder.

At Day 21 after implantation, free bone formation was observed in the ECM group, while no free bone formation was observed in the control group throughout. In the ECM implantation group, bone space was observed among the free bone, sesamoid bone and P2 bones, and soft tissue was found in the bone space (Figure 3, 21 days after implantation). The soft tissue in the bone space decreased over time, and the bone space eventually became transparent (Figure 4, 33 days after implantation). New cartilage was generated at the edge of the sesamoid bone, and the newly generated cartilage could differentiate into bone through endochondral ossification, increasing the volume of the sesamoid bone (Figure 5, 33 days after implantation). On day 65 after implantation, it was observed that the sesamoid bone enlarged and merged with the free bone into one bone, but the sesamoid bone did not merge with the P2 bone (Figure 6). On day 98 after implantation, it was observed that the bone space between the merged bone and P2 still existed, and the joint-like structure remained unchanged (Figure 7). The bone surfaces on both sides of the bone space were covered with cartilage, and the cartilage edge line was an irregular curve. The surface of the regenerated cartilage has non-transparent soft tissue, and chondrocytes are also distributed in these soft tissues (the "eyeball-shaped" cells are chondrocytes).

### Example 3: ECM normal implantation in P3 removal model

P3 removal model: adult mice were anesthetized, and the P3 bone was completely removed without damaging the P2 bone, and soft tissues such as skin were kept as much as possible.

The ECM granules (prepared as in Example 2) were implanted immediately after P3 removal in ECM group, while no implantation is performed in control group after P3 removal. On day 35 after implantation procedure, the murine digit transparent specimens showed no new P3 bone was generated after P3 removal in the control group (Fig. 8.A), while new P3 bone was generated in the ECM group (Figure 8.B). There was a bone space between the regenerated P3 bone and the P2 bone, and a j oint-like structure was formed between the P2 and the regenerated P3 bone. The P2/P3 joint structure was reconstructed, and the diameter of the distal bone of P2 increased.

### Example 4: ECM normal implantation plus delayed implantation in P3 removal model

Adult mice were subjected to normal implantation with ECM granules in the P3 removal model (see Example 3). At week 2 after P3 removal, the mice were again anesthetized, and the skin at the end of the digit was incised without damaging the bone tissue. The ECM granules (prepared as in Example 2) were implanted again through the skin incision in ECM group, while no implantation is conducted in the control group after the skin incision. On day 35 after the second implantation (i.e., delayed implantation), the murine digit transparent specimens showed no new P3 bone was generated after P3 removal in the control group, while new P3 bone was generated in the ECM group. The regenerated P3 bone mass was greater in Example 4 compared to Example 3, and the bone shape of the regenerated P3 was closer to that of the normal P3 (Figure 8.C). The diameter of the distal bone of P2 increased.

## Claims

1. An inducer for use in joint reconstruction in a subject, comprising extracellular matrix (ECM), preferably comprising extracellular matrix as a main active ingredient, e.g. comprising extracellular matrix as the sole active ingredient.

2. The inducer for use according to claim 1, wherein the use is for joint reconstruction in the case of a joint element defect, more preferably the use is for joint reconstruction in the case of complete mission of at least one joint element.

3. The inducer for use according to claim 1, wherein the joint element defect is a freshly generated joint element defect, including the joint element defect that is freshly generated at the j oint site of an old j oint element defect, or old j oint element defect.

4. The inducer for use according to any of claims 1-3, wherein the extracellular matrix is obtained from an animal tissue and organ, including but not limited to extracellular matrices obtained from small intestine, trachea, bladder, bone, or wherein the extracellular matrix is of non-animal tissue or organ origin.

5. An inducer for use according to any of claims 1-4, wherein the extracellular matrix is obtained by physical or chemical treatment, including but not limited to: lyophilizing, extruding, pulverizing, degradation, cross-linking, gelation and 3D printing shaping.

6. An inducer for use according to any one of the preceding claims, wherein the inducer further comprises the additional components selected from one or more of an inorganic salt (e.g. an inorganic salt containing calcium, phosphorus, magnesium, etc.), acetates, citrates, trehalose, hydroxyapatite micronised, glucose, sorbitol, or other osteogenesis-promoting elements.

7. An inducer for use according to any one of the preceding claims, wherein the inducer is in the form of granules, e.g. formed by dry granulation or wet granulation.

8. An inducer for use according to claim 7, wherein, in wet granulation, the additional components are added in the powder mixture to be wetted, or in (e.g. dissolved or suspended in) the liquid for wetting; or a part of the additional elements are added in the powder mixture to be wetted and the rest in (e.g. dissolved or suspended in) the liquid for wetting.

9. An inducer for use according to any one of the preceding claims, wherein the inducer is shaped into the shape of a bone to be regenerated.

10. The inducer for use according to any one of the preceding claims, wherein the inducer is for external use, preferably working by implantation into the lesion site of a tissue and is partially or completely absorbed during regeneration process.

11. The inducer for use according to any one of claims 1-10, wherein the inducer is implanted into a joint lesion site of the subject within 1 week from the joint element defect in the subject.

12. The inducer for use according to any one of claims 1-10, wherein the inducer is implanted into a joint lesion site of a subject at any time point during the period of Week 2-12, or Week 2-8, or Week 2-4 from joint element defect in the subject, e.g., after the healing of wound at the joint defect site.

13. The inducer for use according to any one of claims 1-10, wherein the inducer is implanted into a joint lesion site of the subject within one week from the joint element defect in the subject, and is implanted into a joint lesion site of the subject at any time point during the period of Week 2-12, or Week 2-8, or Week 2-4 from joint element defect in the subject, e.g., after the healing of wound at the j oint defect site.

14. A kit of parts, including two containers, wherein the first container comprises an inducer as defined in any one of claims 1-10 for implantation into a joint defect site of a subject within one week from the joint element defect in the subject, and the second container comprises an inducer as defined in any one of claims 1-10 for implantation into a joint lesion site of the subject at any time point during the period of Week 2-12, or Week 2-8, or Week 2-4 from joint element defect in the subject, e.g., after the healing of wound at the joint defect site.
